# EUROPEAN PATENT APPLICATION

(11) **EP 0 754 465 A1**
(43) Date of publication of application: **22.01.1997**
(21) Application number: 95913357.0
(22) Date of filing: 28.03.1995
(51) Int. Cl.: A61K 47/38

(54) **PROCESS FOR PRODUCING SUSTAINED-RELEASE TABLETS AND ENTERIC TABLETS**

(30) Priority: 01.04.1994 JP 85311/94
(71) Applicant: TSUMURA & CO., Chuo-ku, Tokyo 103 (JP)
(72) Inventor: NAKAI, Yoichiro Tsumura & Co., Ibaraki 300-11 (JP); NAKAI, Yoshinobu Tsumura & Co., Ibaraki 300-11 (JP)
(74) Representative: Woods, Geoffrey Corlett
(86) International application number: JP9500577
(87) International publication number: WO9526753

(57) **Abstract**

The invention relates to a process for producing sustained release tablets by mixing water-insoluble cellulose or derivatives thereof with a drug and a wax, then tableting the mixture into tablets, and thermally treating the tablets, as well as to a process for producing sustained release tablets and enteric tablets by mixing water-insoluble cellulose or derivatives thereof with a drug and a wax, then thermally treating the mixture, and tableting it into tablets.

According to the processes of the invention, there can be produced sustained release tablets for releasing a drug therefrom sustainedly at a nearly constant or arbitrary rate with the efficacy of the drug long-lasting, as well as enteric tablets for releasing a drug not in the stomach but in the intestine.

## Description

### TECHNICAL FIELD

The present invention relates to a process for producing sustained release tablets and enteric tablets. In particular, the present invention relates to a process for producing wax-matrix tablets for releasing a drug therefrom sustainedly at a nearly constant or arbitrary rate, or enterally depending on pH, by controlling the dissolution ability of the tablets using a wax and additives.

### BACKGROUND ART

Sustained release preparations are those excellent in effectiveness, safety etc., having long-lasting efficacy by sustainedly releasing their drug, and as compared with a conventional rapidly release preparation, the frequency of their administration into a patient can be reduced and the concentration of their drug with side effects or toxicity if any can be regulated within certain levels in blood to reduce their side effects or toxicity.

The conventional sustained release preparations can be divided into 3 groups: sparse tab type tablets prepared by tableting a combination of granules whose active ingredient is released at different rates; coating type tablets prepared by applying a hydrophobic coating onto tablets; and matrix-type tablets prepared by tableting a combination of an active ingredient with a wax or a water-soluble polymer.

For sparse tab type tablets, Japanese Patent LOP Publication No. 282323/1990 discloses their production by tableting a combination of granules coated with water-soluble cellulose ether and granules coated further with a wax on the water-soluble cellulose ether, but the disadvantage of this process was that it needs cumbersome production steps.

For coating type tablets, their production by coating tablets with ethylcellulose etc. is known. However, the disadvantage of this production is that the permeability of their coating varies depending numerous factors at the time of prescription, production, storage etc.

For matrix-type tablets using a wax, Japanese Patent LOP Publication No. 14091/1981 discloses the production of matrix-type sustained release tablets by tableting a powder of drug previously treated with a wax.

For matrix-type tablets using a water-soluble polymer, Japanese Patent LOP Publication No. 174311/1983 discloses the production of gel-matrix-type sustained release tablets by tableting a combination of a drug with hydroxypropylmethylcellulose (HPMC) which may further contain mixed base materials such as methylcellulose (MC).

However, these matrix-type tablets will show a drop in concentration gradient or dissolution rate in the last half of dissolution if a wax or HPMC is added in a larger amount. On the other hand, if a wax or HPMC is added in a smaller amount, they fail to play a role as sustained release tablets because they will release their drug at once for their disintegration at the initial stage of dissolution.

To solve these problems, another kind of tablet was devised by combining wax-matrix-type with gel-matrix-type, and Japanese Patent LOP Publication No. 282814/1991 discloses the production of such tablets by mixing nonionic water-soluble cellulose ether, a wax and a drug, followed by direct tableting and subsequent thermal treatment thereof.

However, the tablets prepared in this manner will release their drug from the gel matrix layer (e.g. HPMC), and therefore changes in the gel layer, caused by fluctuations in the movement of the intestinal tract, should be taken into consideration.

A further disadvantage of such processes is that the prescription of tablets should be changed for use of highly water-soluble or slightly water-soluble drugs.

Another disadvantage of conventional processes for producing enteric preparations by coating tablets with e.g. cellulose acetate phthalate insoluble in an acid but soluble in a weak base is that the resulting enteric coating is liable to vary widely in qualities.

### DISCLOSURE OF INVENTION

As a result of their eager study, the present inventors have successfully arrived at a process for producing sustained release tablets and enteric tablets free of the above-described problems by mixing water-insoluble cellulose or derivatives thereof with a wax and a drug, then tableting the powder mixture thus obtained into tablets, followed by thermal treatment thereof, or thermally treating said powder mixture, followed by tableting thereof into tablets.

That is, the present invention encompasses:
(1) A process for producing sustained release tablets and enteric tablets, which comprises mixing water-insoluble cellulose or derivatives thereof with a drug and a wax, then tableting the mixture into tablets, and thermally treating the tablets.
(2) The process according to (1) above, wherein the water-insoluble cellulose or derivatives thereof and the drug are processed by a mixing and grinding method and then mixed with the wax.
(3) The process according to (1) or (2) above, wherein a disintegrator is added.
(4) The process according to any one of (1) to (3) above, wherein the thermal treatment is carried out in a high-frequency heater.
(5) The process according to any one of (1) to (4) above, wherein the mixing ratio of wax in the tablets is in the range of 20 to 50 % by weight.
(6) A process for producing sustained release tablets and enteric tablets, which comprises mixing water-insoluble cellulose or derivative thereof with a drug and a wax, then thermally treating the mixture, and tableting it into tablets.
(7) The process according to (6) above, wherein the water-insoluble cellulose or derivatives thereof and the drug are processed by a mixing and grinding method and then mixed with the wax.
(8) The process according to (6) or (7) above, wherein a disintegrator is added.
(9) The process according to any one of (6) to (8) above, wherein the thermal treatment is carried out in a high-frequency heater.
(10) The process according to any one of (6) to (9) above, wherein the mixing ratio of wax in the tablets is in the range of 20 to 50 % by weight.

Hereinafter, the processes (1) to (10) are referred to collectively as the process of the present invention.

Although the drug used in the process of the present invention is not particularly limited, mention can be made of ethenzamide, diclofenac sodium, theophylline, acetaminophen, indometacin, nifedipine, diltiazem hydrochloride, fluorouracil, aspirin, phenacetin, molsidomine, niceritrol, pentoxifylline, propranolol hydrochloride, prednisolone, cloperastine, chlorpheniramine maleate etc.

The amount of drug in tablets is preferably 1 to 40 % by weight, more preferably 2 to 30 % by weight.

The water-insoluble cellulose or derivatives thereof used in the present invention are cellulose or nonionic or ionic cellulose derivatives, and they are not particularly limited if they are water-insoluble.

Examples are crystalline cellulose, ethylcellulose, low-substituted hydroxypropylcellulose, carmellose, carmellose calcium, crosscarmellose sodium, carboxymethylethylcellulose, cellulose acetate phthalate, etc. They are used singly or in combination. In particular, crystalline cellulose is preferably used.

Out of the above water-insoluble cellulose derivatives, ethylcellulose has been used as a sustained release coating agent where a desired control dissolution rate can be attained by changing its amount relative to the amount of solvent in a coating step. However, its employment in the present invention differs from such known employment in both object and effect.

Although the employment of ethyl cellulose as a coating agent causes remarkably decreased bioavailability sometimes depending on drug, the employment thereof in the process of the present invention is free of this problem.

The amount of water-insoluble cellulose or derivatives thereof in tablets is preferably 25 to 80 % by weight, more preferably 40 to 70 % by weight.

The wax used in the process of the present invention may be any wax insofar as it is insoluble or slightly soluble in water and its melting point ranges 50 to 100°C.

Examples are natural wax such as carnauba wax, beeswax, white beeswax, etc.; higher aliphatic acids such as stearic acid etc.; paraffins such as paraffin wax, microwax, microcrystalline wax, etc.; various hydrogenated oils, such as beef tallow hydrogenated oil, hydrogenated castor oil, soybean hydrogenated oil, hydrogenated rape seed oil etc.; and higher alcohols such as cetyl alcohol, stearyl alcohol, etc.

Suitable wax is selected depending on the properties of drug used, the amount of wax, desired dissolution time, etc.

The amount of wax in tablets is selected depending on the properties of drug used, desired duration time etc. However, if a too large amount of wax is used in tablets, it is hard to dissolve the drug at a constant rate from the tablets. Therefore, the amount of wax in the tablets is preferably 60 % by weight or less, more preferably in the range of 20 to 50 % by weight.

In the process of the invention, any thermal treatment can be adopted insofar as the tablets or mixture can be heated to about the melting point of wax used.

If a powder mixture, prepared by mixing water-insoluble cellulose or derivatives thereof with a wax and a drug, is first tableted and then treated thermally, the production unit used is specifically a ventilating oven, a high-frequency heater, etc. In particular, a high-frequency heater is preferably used for its easy operation etc.

If a high-frequency heater is used, e.g. a microwave oven is used for thermal treatment over a suitable time depending on the kinds and amounts of drug, additives and wax in the tablets.

If a powder mixture, prepared by mixing water-insoluble cellulose or derivatives thereof with a wax and a drug, is first treated thermally and then tableted, the production unit used is specifically a mixing apparatus, a kneader, or a stirrer, equipped with a heater.

The mixing and grinding method referred to in the process of the present invention is a technique involving mixing water-insoluble cellulose or derivatives thereof with a drug and then mixing and grinding the mixture in a vibration rod mill etc., as described in Japanese Patent LOP Publication No. 22138/1978.

By this technique, drug crystals are made amorphous to permit significantly rapid dissolution of drug from the powdered mixture, to increase absorption rate, and to prevent volatile drug from sublimating (Yakugaku Zasshi, 105, 801 (1985)).

Some water-insoluble cellulose or derivatives thereof used in the present invention, e.g. crystalline cellulose, low-substituted hydroxypropylcellulose, carmellose calcium, crosscarmellose sodium, and carmellose, can also function as disintegrators, but other disintegrators can also be used if required. Such disintegrators are not particularly limited, including partially pregelatinised starch, carboxymethyl starch sodium, carmellose sodium, various kinds of starch, and hydroxypropyl starch.

The amount of disintegrator is selected depending on a desired dissolution rate, since the disintegration ability varies depending on disintegrator type, and pH.

For example, ionic disintegrators are used for enteric dissolution. Some water-insoluble cellulose or derivatives thereof used in the present invention, e.g. crosscarmellose sodium and carmellose calcium, can also function as said ionic disintegrators. However, other ionic disintegrators such as carboxymethyl starch sodium may be used if necessary.

In the present invention, the drug after mixed and ground together with water-insoluble cellulose or derivatives thereof shows negligible dissolution rate of the drug, and its dissolution rate can be controlled arbitrarily with a suitable amount of disintegrator. Hence, tablets with various dissolution patterns can be obtained as e.g. an enteric preparation using a combination of suitable type and amount of disintegrator.

In the present invention, a lubricant such as magnesium stearate can further be added in a suitable amount to the tablets if the tableting step is hindered by e.g. sticking readily occurring for poor powder fluidity.

A lubricant such as magnesium stearate is added in an amount of about 1 to 2 % according to a conventional method.

### BEST MODE FOR CARRYING OUT THE INVENTION

Hereinafter, the present invention is described in more detail by Experimental Examples and Examples, which however are not intended to limit the scope of the present invention.

### Experimental Example 1

In this example, the tablets of the present invention were prepared using different treatments of a drug and water-insoluble cellulose or derivatives thereof and they were examined for their dissolution ability.

The following ingredients were mixed in the ratios shown in Table 1 in a mortar for 5 minutes. Each powder mixture was tableted in a compressing machine (manufactured by ERWEKA) and then thermally treated whereby cylindrical flat tablets of 6 mm diameter with a hardness of 9 to 11 kg, each weighing 100 mg, were manufactured.

**Table 1**

| Example 1 | Example 2 | Example 3 | Ratio (%) |
|---|---|---|---|
| crystalline cellulose | crystalline cellulose G | | 57 |
| ethenzamide | ethenzamide | | 3 |
| | | ground mixture | 60 |
| carnauba wax | carnauba wax | carnauba wax | 29 |
| crystalline cellulose G | crystalline cellulose G | crystalline cellulose G | 10 |
| magnesium stearate | magnesium stearate | magnesium stearate | 1 |

In Table 1, "crystalline cellulose G" was prepared by grinding crystalline cellulose in a vibration rod mill (manufactured by C.M.T. Ltd.), and "ground mixture" was prepared by mixing crystalline cellulose with a drug (ethenzamide in this case) at a ratio of 57:3 and then grinding the mixture in a vibration rod mill (manufactured by C.M.T. Ltd.).

The tablets thus manufactured were examined for their dissolution ability under the following conditions, and the results are shown in Table 2.
- Test method:: Japanese Pharmacopoeia (12th revised edition), Puddle method.
- Test solution:: Purified water, 900 ml, 37 °C.
- Number of Puddle Rotations:: 100 rpm.

**Table 2**

| | dissolution ratio (%) with time (hr) | | | | | | | |
|---|---|---|---|---|---|---|---|---|
| | 0 | 0.5 | 1 | 2 | 4 | 8 | 12 | 24 |
| Example 1 | 0.0 | 11.8 | 20.2 | 31.3 | 45.9 | 63.1 | 73.9 | 92.8 |
| Example 2 | 0.0 | 7.2 | 10.2 | 14.2 | 20.3 | 31.0 | 42.7 | 72.3 |
| Example 3 | 0.0 | 7.2 | 9.9 | 13.6 | 19.4 | 30.9 | 45.7 | 73.3 |

As is evident from Table 2, the drug and water-insoluble cellulose or derivatives thereof used in the process of the present invention may be simply mixed with each other, or otherwise their mixture may be ground, depending on desired dissolution ability.

### Experimental Example 2

In this example, the tablets having different hardness were prepared and examined for their dissolution ability.

The following ingredients were mixed in the ratio shown in Table 1 for Example 3 in a mortar for 5 minutes. The powder mixture was tableted under 3 different compressing pressures in a compressing machine (manufactured by ERWEKA) to give tablets with a hardness of 3 kg, 8 kg or 11 kg. These tablets were thermally treated whereby cylindrical flat tablets of 6 mm diameter, each weighing 100 mg, were manufactured.

The tablets thus manufactured were examined for their dissolution ability in the same manner as in Experimental Example 1. The results are shown in Table 3.

**Table 3**

| | dissolution ratio (%) with time (hr) | | | | | | |
|---|---|---|---|---|---|---|---|
| | 0 | 0.5 | 1 | 2 | 4 | 8 | 12 |
| 3 kg | 0.0 | 6.7 | 9.4 | 13.0 | 18.6 | 28.2 | 37.1 |
| 8 kg | 0.0 | 7.2 | 9.9 | 13.6 | 19.4 | 30.9 | 45.7 |
| 11 kg | 0.0 | 6.2 | 9.0 | 12.9 | 19.4 | 31.5 | 46.3 |

As is evident from Table 3, it was confirmed that their dissolution ability is not affected by their tablet hardness given before thermal treatment.

### Experimental Example 3

In Experimental Examples 3 and 4, the tablets were prepared in different embodiments of the present invention and examined for their dissolution ability.

The following ingredients were mixed in the ratios shown in Table 4 in a mortar for 5 minutes. Each powder mixture was first tableted in a compressing machine (manufactured by ERWEKA) and then treated thermally whereby tablets of 8.5 mm diameter each weighing 250 mg were manufactured.

### Experimental Example 4

The following ingredients were mixed in the ratio shown in Table 4 in a mortar for 5 minutes. The powder mixture was first treated thermally and then tableted in a compressing machine whereby tablets of 8.5 mm diameter each weighing 250 mg were manufactured.

**Table 4**

| | Example 4 |
|---|---|
| crystalline cellulose G | 44 |
| diclofenac sodium | 30 |
| carnauba wax | 25 |
| magnesium stearate | 1 |

The tablets manufactured in Experimental Examples 3 and 4 were examined for their dissolution ability under the following conditions. The results are shown in Table 5.
- Test method:: Japanese Pharmacopoeia (12th revised edition), Puddle method.
- Test solution:: Japanese Pharmacopoeia second solution (pH = 6.8), 900 ml, 37 °C.
- Number of Puddle Rotations:: 100 rpm.

**Table 5**

| | dissolution ratio (%) with time (hr) | | | | | | | |
|---|---|---|---|---|---|---|---|---|
| | 0 | 0.5 | 1 | 2 | 4 | 8 | 12 | 24 |
| Example 3 | 0.0 | 11.0 | 14.0 | 19.0 | 29.1 | 52.9 | 67.7 | 87.9 |
| Example 4 | 0.0 | 9.1 | 12.4 | 18.1 | 29.5 | 58.3 | 75.9 | 96.1 |

As is evident from Table 5, it was confirmed that the powder mixture may be first tableted and then treated thermally, or alternatively the powder mixture may be first treated thermally and then tableted. Hence, either of the 2 processes may be used depending on available apparatuses.

### Experimental Example 5

In this example, the tablets were prepared using different amounts of wax and examined for their dissolution ability.

The tablets were manufactured in the same manner as in Experimental Example 1 except that the ratios shown in Table 6 were used.

**Table 6**

| | Ex. 3 | Ex. 5 | Ex. 6 | Ex. 7 | Ex. 8 | Ex.9 | Ex. 10 | Com. Ex. 1 |
|---|---|---|---|---|---|---|---|---|
| ground mixture | 60 | 60 | 60 | 40 | 40 | 40 | 40 | 60 |
| carnauba wax | 29 | 19 | 9 | 29 | 39 | 49 | 59 | 0 |
| crystalline cellulose G | 10 | 20 | 30 | 30 | 20 | 10 | 0 | 39 |
| magnesium stearate | 1 | 1 | 1 | 1 | 1 | 1 | 1 | 1 |
| Ex.: Example Com. Ex.: Comparative Example | | | | | | | | |

The tablets thus manufactured was examined for their dissolution ability in the same manner as in Experimental Example 1. The results are shown in Table 7.

**Table 7**

| | dissolution ratio (%) with time (hr) | | | | | | |
|---|---|---|---|---|---|---|---|
| | 0 | 0.5 | 1 | 2 | 4 | 8 | 12 |
| Example 3 | 0.0 | 7.2 | 9.9 | 13.6 | 19.4 | 30.9 | 45.7 |
| Example 5 | 0.0 | 7.2 | 10.2 | 14.1 | 21.4 | 35.7 | 53.0 |
| Example 6 | 0.0 | 7.5 | 11.0 | 17.2 | 34.2 | 62.9 | 75.6 |
| Example 7 | 0.0 | 7.1 | 10.5 | 15.4 | 22.5 | 36.8 | 53.6 |
| Example 8 | 0.0 | 7.2 | 10.7 | 14.9 | 21.2 | 31.9 | 43.5 |
| Example 9 | 0.0 | 5.6 | 8.1 | 12.2 | 17.1 | 25.2 | 33.0 |
| Example 10 | 0.0 | 5.7 | 8.9 | 13.0 | 19.5 | 30.6 | 42.9 |
| Comparative Example 1 | 0.0 | 61.7 | 80.1 | 91.5 | 94.9 | 95.9 | 96.8 |

As shown in Table 7, the amount of wax in the tablets can be selected depending on the properties of drug used, desired duration time, etc., but if wax is used in a larger amount, it is hard to attain a constant dissolution rate of the drug from the tablets. Therefore, the wax in the tablets is preferably 60 % by weight or less, more preferably in the range of 20 to 50 %.

### Example 6

In this example, the tablets were prepared using high-frequency heating for different periods of time and examined for their dissolution ability.

The following ingredients were mixed in a mortar for 5 minutes in the ratio shown above in Table 1 for Example 3. The powder mixture was tableted in a compressing machine (manufactured by ERWEKA) and then subjected to high-frequency heating in a microwave oven (High Cooker R528, rated high-frequency output: 500 W, oscillation frequency: 2450 MHz, manufactured by Sharp Corporation) for 0, 1 minute or 2 minutes whereby cylindrical flat tablets of 6 mm diameter each weighing 100 mg were manufactured.

The tablets thus prepared were examined for their dissolution ability in the same manner as in Experimental Example 1. The results are shown in Table 8.

**Table 8**

| | dissolution ratio (%) with time (hr) | | | | | | |
|---|---|---|---|---|---|---|---|
| | 0 | 0.5 | 1 | 2 | 3 | 4 | 5 |
| 0 min. | 0.0 | 10.6 | 18.3 | 30.9 | 40.9 | 48.3 | 54.1 |
| 1 min. | 0.0 | 7.2 | 9.9 | 13.6 | 16.5 | 19.4 | 22.0 |
| 2 min. | 0.0 | 7.6 | 10.6 | 14.7 | 18.0 | 21.3 | 25.0 |

As is evident from Table 8, it was confirmed that high-frequency heating for 1 minute is sufficient for the tablets shown in Table 1 for Example 3.

### Experimental Example 7

In this example, the tablets were prepared using different kinds and amounts of disintegrator and examined for their dissolution ability.

The tablets were manufactured in the same manner as in Experimental Example 1 except that the ratios shown in Table 9 were used.

**Table 9**

| | Ex. 11 | Ex. 12 | Ex. 13 | Ex. 14 | Ex. 15 |
|---|---|---|---|---|---|
| ground mixture | 60.0 | 60.0 | 60.0 | 60.0 | 60.0 |
| carnauba wax | 29.0 | 29.0 | 29.0 | 29.0 | 29.0 |
| crystalline cellulose G | 5.0 | 0 | 9.0 | 7.5 | 5.0 |
| low-substituted hydroxypropylcellulose | 5.0 | 10.0 | 0 | 0 | 0 |
| crosscarmellose sodium | 0 | 0 | 1.0 | 2.5 | 5.0 |
| magnesium stearate | 1.0 | 1.0 | 1.0 | 1.0 | 1.0 |

The tablets thus manufactured were examined for their dissolution ability under the following conditions.
- Test method:: Japanese Pharmacopoeia (12th revised edition), Puddle method.
- Test solutions:: Japanese Pharmacopoeia first solution (pH = 1.2), 900 ml, 37 °C
: Japanese Pharmacopoeia second solution (pH = 6.8), 900 ml, 37°C
- Number of Puddle Rotations:: 100 rpm.

The results obtained using the Japanese Pharmacopoeia first and second solutions are shown in Tables 10 and 11, respectively.

**Table 10**

| | dissolution ratio (%) with time (hr) | | | | | | |
|---|---|---|---|---|---|---|---|
| | 0 | 0.5 | 1 | 2 | 4 | 8 | 12 |
| Example 3 | 0.0 | 6.5 | 9.2 | 13.3 | 19.2 | 29.2 | 39.4 |
| Example 11 | 0.0 | 8.7 | 11.8 | 16.8 | 25.2 | 45.8 | 70.7 |
| Example 12 | 0.0 | 12.9 | 21.1 | 38.5 | 81.8 | 94.7 | 95.4 |
| Example 13 | 0.0 | 7.3 | 10.1 | 14.1 | 20.3 | 30.2 | 42.1 |
| Example 14 | 0.0 | 7.5 | 10.3 | 15.1 | 21.9 | 33.1 | 47.8 |
| Example 15 | 0.0 | 10.7 | 15.2 | 21.1 | 29.7 | 45.0 | 62.0 |

**Table 11**

| | dissolution ratio (%) with time (hr) | | | | | | |
|---|---|---|---|---|---|---|---|
| | 0 | 0.5 | 1 | 2 | 4 | 8 | 12 |
| Example 3 | 0.0 | 6.6 | 9.3 | 13.2 | 18.7 | 29.1 | 40.7 |
| Example 11 | 0.0 | 9.4 | 12.8 | 17.9 | 26.6 | 46.8 | |
| Example 12 | 0.0 | 11.7 | 18.4 | 33.1 | 70.5 | 95.4 | 96.3 |
| Example 13 | 0.0 | 8.0 | 11.0 | 16.0 | 24.9 | 46.7 | 74.2 |
| Example 14 | 0.0 | 13.4 | 25.5 | 52.7 | 90.3 | 96.4 | 97.2 |
| Example 15 | 0.0 | 45.3 | 72.0 | 90.0 | 91.0 | 92.1 | |

As can be seen from Tables 10 and 11, low-substituted hydroxypropylcellulose is pH-independent and brings about constant releasing ability due to its constant disintegrability not affected by the pH of the dissolution solution. On the other hand, crosscarmellose sodium is pH-dependent and shows disintegrability varying dependent on the pH of the dissolution solution. Hence, with crosscarmellose sodium, the release of the drug can be inhibited under acidic conditions while it is readily released under weakly acidic or neutral conditions, and the enteric dissolution of the drug can be controlled.

Because a disintegrator shows different disintegration power depending on type of disintegrator and pH for dissolution, the amount of disintegrator is selected depending on desired dissolution.

### Experimental Example 8

In this example, the tablets were examined for their dissolution ability by use of different numbers of puddle rotations in a dissolution test.

The tablets of Example 3 shown in Table 1 above were examined under the following conditions.
- Test method:: Japanese Pharmacopoeia (12th revised edition), Puddle method.
- Test solution:: Purified water, 900 ml, 37°C.
- Number of Puddle Rotations:: 50 rpm.
: 100 rpm.
: 200 rpm.

The results are shown in Table 12.

**Table 12**

| | dissolution ratio (%) with time (hr) | | | | | | |
|---|---|---|---|---|---|---|---|
| | 0 | 0.5 | 1 | 2 | 4 | 8 | 12 |
| 50 rpm | 0.0 | 6.8 | 9.3 | 13.0 | 18.5 | 28.6 | 40.6 |
| 100 rpm | 0.0 | 7.2 | 9.9 | 13.6 | 19.4 | 30.9 | 45.7 |
| 200 rpm | 0.0 | 6.5 | 9.3 | 13.4 | 19.2 | 29.4 | 42.2 |

As can be seen from Table 12, the tablets obtained in the process of the present invention show a dissolution ability not affected by rotation speeds.

As described above, the dissolution ability of the tablets manufactured in the present process is not changed regardless of their hardness or affected by rotation speeds. Further, their dissolution rate can be controlled by adding additives such as disintegrator etc., so that they can be pH-independent tablets or enteric tablets dissolving depending on pH.

### Examples 16 and 17

The following ingredients were mixed in the ratios shown in Table 13 in a mortar for 5 minutes. Each powder mixture was tableted in a compressing machine (manufactured by ERWEKA) and thermally treated whereby cylindrical flat tablets with a hardness of 9 to 11 kg, each weighing 100 mg, were manufactured.

**Table 13**

| Example 16 | Example 17 | Ratio (%) |
|---|---|---|
| crystalline cellulose G | | 57 |
| phenacetin | | 3 |
| | ground mixture | 60 |
| carnauba wax | carnauba wax | 29 |
| crystalline cellulose G | crystalline cellulose G | 10 |
| magnesium stearate | magnesium stearate | 1 |

In Table 13, "ground mixture" was prepared by mixing crystalline cellulose with phenacetin at a ratio of 57:3 and grinding the mixture in a vibration rod mill (manufactured by C.M.T. Ltd.).

The tablets thus manufactured were examined for their dissolution ability under the following conditions. The results are shown in Table 14.
- Test method:: Japanese Pharmacopoeia (12th revised edition), Puddle method.
- Test solution:: Purified water, 900 ml, 37°C.
- Number of Puddle Rotations:: 100 rpm.

**Table 14**

| | dissolution ratio (%) with time (hr) | | | | | | |
|---|---|---|---|---|---|---|---|
| | 0 | 0.5 | 1 | 2 | 4 | 8 | 12 |
| Example 16 | 0.0 | 4.0 | 5.8 | 8.7 | 13.1 | 21.3 | 31.9 |
| Example 17 | 0.0 | 4.3 | 6.4 | 9.3 | 13.8 | 21.6 | 29.6 |

### Examples 18 and 19

The following ingredients were mixed in the ratios shown in Table 15 in a mortar for 5 minutes. Each powder mixture was tableted in a compressing machine (manufactured by ERWEKA) and thermally treated whereby cylindrical flat tablets with a hardness of 9 to 11 kg, each weighing 100 mg, were manufactured.

**Table 15**

| Example 18 | Example 19 | Ratio (%) |
|---|---|---|
| crystalline cellulose G | | 54 |
| ethenzamide | | 6 |
| | ground mixture | 60 |
| carnauba wax | carnauba wax | 29 |
| crystalline cellulose G | crystalline cellulose G | 10 |
| magnesium stearate | magnesium stearate | 1 |

In Table 15, "ground mixture" was prepared by mixing crystalline cellulose with ethenzamide at a ratio of 54:6 and grinding the mixture in a vibration rod mill (manufactured by C.M.T. Ltd.).

The tablets thus manufactured were examined for their dissolution ability under the following conditions. The results are shown in Table 16.
- Test method:: Japanese Pharmacopoeia (12th revised edition), Puddle method.
- Test solution:: Purified water, 900 ml, 37°C.
- Number of Puddle Rotations:: 100 rpm.

**Table 16**

| | dissolution ratio (%) with time (hr) | | | | | | |
|---|---|---|---|---|---|---|---|
| | 0 | 0.5 | 1 | 2 | 4 | 8 | 12 |
| Example 18 | 0.0 | 3.6 | 6.3 | 10.5 | 15.2 | 27.0 | 39.7 |
| Example 19 | 0.0 | 4.7 | 7.4 | 11.4 | 17.2 | 26.6 | 39.9 |

### INDUSTRIAL APPLICABILITY

According to the process of the present invention, it is possible to produce sustained release tablets for releasing a drug therefrom sustainedly at a nearly constant or arbitrary rate with the efficacy of the drug long-lasting, as well as enteric tablets for releasing a drug in the intestine but not in the stomach. Hence, the present invention is useful in the field of pharmaceutical manufacturing.

## Claims

1. A process for producing sustained release tablets and enteric tablets, which comprises mixing water-insoluble cellulose or derivatives thereof with a drug and a wax, then tableting the mixture into tablets, and thermally treating the tablets.

2. The process according to claim 1, wherein the water-insoluble cellulose or derivatives thereof and the drug are processed by a mixing and grinding method and then mixed with the wax.

3. The process according to claim 1, wherein a disintegrator is added.

4. The process according to claim 1, wherein the thermal treatment is carried out in a high-frequency heater.

5. The process according to claim 1, wherein the mixing ratio of wax in the tablets is in the range of 20 to 50 % by weight.

6. A process for producing sustained release tablets and enteric tablets, which comprises mixing water-insoluble cellulose or derivative thereof with a drug and a wax, then thermally treating the mixture, and tableting it into tablets.

7. The process according to claim 6, wherein the water-insoluble cellulose or derivatives thereof and the drug are processed by a mixing and grinding method and then mixed with the wax.

8. The process according to claim 6, wherein a disintegrator is added.

9. The process according to claim 6, wherein the thermal treatment is carried out in a high-frequency heater.

10. The process according to claim 6, wherein the mixing ratio of wax in the tablets is in the range of 20 to 50 % by weight.
